# EUROPEAN PATENT SPECIFICATION

(11) **EP 1 534 228 B1**
(45) Date of publication and mention of the grant of the patent: **14.06.2006**
(21) Application number: 03793753.9
(22) Date of filing: 21.08.2003
(51) Int. Cl.: A61Q 9/02

(54) **SHAVING COMPOSITION**
RASIERZUSAMMENSETZUNG
COMPOSITION DE RASAGE

(30) Priority: 05.09.2002 US 408241 P
(43) Date of publication of application: 01.06.2005
(73) Proprietor: UNILEVER PLC, London EC4P 4BQ (GB); UNILEVER N.V., 3013 AL Rotterdam (NL)
(72) Inventor: GOLDBERG, Jessica, Weiss, Trumbull, CT 06611 (US); MASSARO, Michael, Trumbull, CT 06611 (US); BHATT, Darshna, Unilever Home & Personal Care USA, IL 60008 (US)
(74) Representative: Mulder, Cornelis Willem Reinier
(86) International application number: PCT/EP2003/009383
(87) International publication number: WO 2004/022017

(56) References cited:
- EP-A- 0 285 574
- EP-A- 0 796 611
- US-A- 3 959 160
- PATENT ABSTRACTS OF JAPAN vol. 012, no. 387 (C-536), 14 October 1988 (1988-10-14) & JP 63 132817 A (SHISEIDO CO LTD), 4 June 1988 (1988-06-04)

## Description

The invention relates to shaving compositions delivered as aerosols exhibiting enhanced foaming qualities.

Shaving is an inherently abrasive treatment of the skin. Cosmetic foams have been created to lubricate the cutting process. Lubrication markedly reduces the trauma induced by a razor.

Emollient ingredients of shaving creams are substantial contributors to reduction of irritation caused by the razor action.

Emollients have long been incorporated into cleansing compositions. For instance, U.S. Patent 5,002,680 (Schmidt et al.) discloses a mild skin-cleansing aerosol mousse. Nice skin feel after washing is imparted to the skin through a combination of mild surfactants, skinfeel polymers and high levels of moisturizing emollients. Similar skin cleansing compositions have been reported in U.S. Patent 6,407,044 B2 (Dixon) wherein a hydrocarbon propellant system was used to generate a cleansing foam.

A problem with use of high levels of emollients is that they depress foam formation often even collapsing the bubbles. Systems are needed which foam well despite the presence of high levels of emollient oils.

EP 0 285 574 (Cavazza) relates to a soap free, non-lathering, moisturising shaving cream comprising a long chain alcohol selected from stearyl, lauryl, cetyl or myristyl alcohol; an anionic, amphoteric or quaternary surfactant and/or an emulsifying agent, where if a quaternary is present the anionic is absent; a wetting agent selected from glycerol, propylene glycol, sorbitol and polyethylene glycol and an emollient selected from mineral oil and vaseline.

EP 0 796 611 (Lee et al.) relates to a hair styling mousse composition including a water-soluble film forming resin, an amphoteric surfactant and a propellant which is a mixture of a hydrocarbon and a C₁ - C₄ dialkyl ether in a ratio of from 10:1 to 1:4.

In a first aspect, there is provided a shaving cream composition which includes:
(i) from 1 % to 20 % of a propellant comprising dimethyl ether and hydrocarbons in a relative weight ratio of 5:1 to 1:5;
(ii) from 0.5 % to 25 % of an anionic surfactant;
(iii) from 0.05 to 20 % of a C₁₂-C₂₄ fatty alcohol;
(iv) from 5 % to 40 % of a hydrophobic emollient;
wherein a total weight ratio of all hydrophobic emollients to all anionic surfactants present in the composition ranges from 50:1 to greater than 1:1.

Now it has been found that a combination of factors can influence generation of a rich, long lasting shaving foam despite the presence of very high emollient levels. One factor is utilization of fatty alcohols which were found to interact with the surfactants to create small sustainable bubbles. Another factor is the use of dimethyl ether as at least one component of the propellant system. Fatty alcohol, dimethyl ether and the emollient all combine to provide a rich lasting foam that also delivers substantial emollient to the interface between skin and razor.

Accordingly, a first element of the present invention is that of a propellant. The propellant is present in amounts from 1 % to 20 %, preferably from about 3 % to about 15 %, optimally from about 5 % to about 10 % by weight of the composition. The propellant is a mixture of dimethyl ether and a C₃-C₆ hydrocarbon. Suitable hydrocarbons include n-butane, isobutane, n-propane, isopropane, n-pentane, isopentane and mixtures thereof. Amounts of the dimethyl ether and total hydrocarbon are present in a relative weight ratio ranging from about 5:1 to about 1:5, preferably from about 4:1 to about 1:4, optimally from about 2:1 to about 1:2, and most optimally from about 1:1 to about 1:1.5.

Another component of the present invention is that of one or more anionic surfactants. These will be present in amounts from 0.5 % to 25 %, preferably from about 1 % to about 15 %, more preferably from about 3 % to about 10 %, optimally from about 4 % to about 8 % by weight of the composition.

Anionic surfactants useful herein include: acyl isethionates, acyl sarcosinates, alkylglycerylether sulfonates, alkyl sulfates, acyl lactylate, methylacyl taurates, paraffin sulfonates, linear alkyl benzene sulfonates, N-acyl glutamates, alkyl sulfosuccinates, alpha sulfo fatty acid esters, alkyl ether carboxylates, alkyl phosphate esters, ethoxylated alkyl phosphate esters, alpha olefin sulphates, the alkyl ether sulfates (with 1 to 12 ethoxy groups) and mixtures thereof, wherein said surfactants contain C8 to C22 alkyl chains and wherein the counterion is preferably selected from the group consisting of Na, K, NH₄, N(CH₂CH₂OH)₃. Most preferred are lauryl ether sulfates.

Although soaps (i.e. salts of C₁₀-C₂₂ fatty acids) are often used in shaving formulations, these materials are relatively irritating to the skin. For purposes of this invention, amounts of the soap advantageously is limited to no more than about 10 %, ordinarily no more than about 2 %, and preferably less than 1 % or especially less than 0.1 % by weight of the composition. Similarly, unsaponified soaps which are known as C₁₀-C₂₄ fatty acids advantageously can also be avoided because of their acidity and foam inhibiting properties, particularly stearic acid. Levels of fatty acid, particularly stearic acid, is best held to less than 1 %, preferably less than 0.8 %, optimally less than 0.1 % by weight of the composition. Under some circumstances, an amine neutralized fatty acid such as triethanolammonium stearate can be employed, particularly at levels from about 0.2 % to about 1 % by weight.

Amphoteric surfactants may be included in compositions of this invention. Amounts may range from about 0.5 % to about 15 %, preferably from about 1 % to about 10 %, optimally from about 3 % to about 6 % by weight. Illustrative of the amphoteric surfactant are cocoamidopropyl betaine, lauroamphoacetates and diacetates, alkyl dimethylamine oxides and combinations thereof. Particularly preferred are combinations of anionic and amphoteric surfactants, especially present in relative weight ratios of total anionic to total amphoteric surfactant ranging from about 10:1 to about 1:1, preferably about 3:1 to about 1.5:1.

The pH of the composition may range from about 4.5 to about 12.5. However, the pH is preferably near neutral and may range from about 6.5 to about 9.5, preferably from about 6.7 to about 8.5, optimally from about 7 to about 7.5.

Another component of compositions according to this present invention is a C₁₂-C₂₄ fatty alcohol. This material is present in an amount from 0.05 % to 20 %, preferably from about 0.2 % to about 8 %, more preferably from about 0.5 % to about 5 %, optimally from about 1 % to about 3 % by weight of the composition.

Illustrative fatty alcohols include stearyl alcohol, lauryl alcohol, myristyl alcohol, cetyl alcohol, behenyl alcohol and combinations thereof.

Hydrophobic emollients are an important component of the present invention. These materials are present in an amount from 5 % to 40 %, preferably from about 8 % to about 30 %, more preferably from about 10 % to about 25 %, optimally from about 15 % to about 20 % total of hydrophobic emollient by weight of the composition.

Hydrophobic emollients may typically be in the form of natural oils (particularly vegetable oils), synthetic esters, hydrocarbons and silicone oils.

Natural oils include triglycerides such as sunflower seed oil, cottonseed oil, safflower oil, olive oil, rapeseed oil, canola oil, linseed oil, shea nut oil, palm oil, corn oil, babassu oil, peanut oil, sesame oil and combinations thereof. Chemically modified versions of vegetable oils may also be utilized. For instance, suitable may be brominated soybean oil and maleated soybean oil. Lanolin oils are another category of natural oils suitable for the present invention. Particularly useful is lanolin alcohol.

Suitable synthetic esters may include:
(1) Alkenyl or alkyl esters of fatty acids having 10 to 20 carbon atoms. Examples thereof include isopropyl palmitate, isopropyl oleate, methyl palmitate, isoarachidyl neopentanoate, isononyl isonanonoate, oleyl myristate, oleyl stearate, and oleyl oleate.
(2) Wax esters such as beeswax, spermaceti wax and tribehenin wax.
(3) Sterols esters, of which cholesterol fatty acid esters are examples thereof.
(4) Sugar ester of fatty acids such as sucrose polybehenate and sucrose polycottonseedate.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Non-volatile silicone oils useful as an emollient material include polyalkyl siloxanes, polyalkylaryl siloxanes and polyether siloxane copolymers. The essentially nonvolatile polyalkyl siloxanes useful herein include, for example, polydimethyl siloxanes with viscosities of from about 5 x 10⁻⁶ to 0.1 m²/s at 25°C. Among the preferred nonvolatile emollients useful in the present compositions are the polydimethyl siloxanes having viscosities from about 1 x 10⁻⁵ to about 4 x 10⁻⁴ m²/s at 25°C.

Another class of non-volatile silicones are emulsifying and non-emulsifying silicone elastomers. Representative of this category is Dimethicone/Vinyl Dimethicone Crosspolymer available as Dow Corning 9040, General Electric SFE 839, and Shin-Etsu KSG-18. Silicone waxes such as Silwax WS-L (Dimethicone Copolyol Laurate) may also be useful.

Hydrocarbons which are suitable as hydrophobic emollients include petrolatum, mineral oil, C₁₁-C₁₃ isoparaffins, polyalphaolefins, and especially isohexadecane, available commercially as Permethyl 101A from Presperse Inc.

The total weight ratio of all hydrophobic emollients to all anionic surfactants ranges from 50:1 to greater than 1:1, preferably from about 30:1 to about 2:1, optimally from about 5:1 to about 1.5:1.

Hydrophilic emollients may also be included in compositions of this invention. These type are usually polyhydric alcohols which include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of hydrophilic emollient may range anywhere from about 0.5 % to about 30 %, preferably between about 1 % and about 10 % by weight of the composition. When both hydrophobic and hydrophilic emollients are present they may range from about 20:1 to greater than about 1:1 in weight ratio.

Preservatives may also desirably be incorporated into the compositions of this invention to protect against the growth of potentially harmful microorganisms. Preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, imidazolidinyl urea, sodium dehydroacetate and benzyl alcohol.

The preservatives should be selected having regard for the use of the composition and possible incompatibilities between the preservatives and other ingredients in the emulsion. Preservatives are preferably employed in amounts ranging from 0.01 % to 2 % by weight of the composition.

Skin conditioners may be incorporated into compositions of this invention. For instance, these materials can be cationic polysaccharides and particularly cationic guar gums with number average molecular weights ranging from 1000 to 3 million; polymers formed from acrylic and/or methacrylic acid; cationic polymers of dimethyl dialkyl ammonium salts and acrylic acids; cationic homopolymers of dimethyl dialkylammonium salts; cationic polyalkylene and ethoxy polyalkylene imines; polyethylene glycol of molecular weight from 100,000 to 4 million; and combinations thereof. Particularly suitable are sodium polyacrylate, hydroxyethyl cellulose and combinations thereof. Most particularly useful are quaternary materials identified by the CTFA names of polyquaternium-3, polyquaternium-5, polyquaternium-6, polyquaternium-7, polyquaternium-10, polyquaternium-11 and polyquaternium-24.

Most preferred is guar hydroxypropyl trimonium chloride sold as Jaguar C13S by Rhone Poulenc. Amounts of the conditioners may range from about 0.01 % to about 2 %, preferably from about 0.1 % to about 1 %, optimally from about 0.2 %to about 0.8 % by weight of the composition.

Sunscreen actives may be included in the compositions. Particularly preferred are Parsol MCX® , Parsol 1789® and benzophenone-3. Inorganic sunscreen actives may be employed such as microfine titanium dioxide, zinc oxide, polyethylene and various other polymers. Amounts of the sunscreen agents when present may generally range from 0.1 % to 15 %, preferably from 2 % to 8 %, optimally from 3 % to 6 % by weight.

Anti-microbial agents which are intended for deposition onto body surfaces may also be formulated into the compositions. Illustrative are the aluminum salts including aluminum chlorohydrate, aluminum zicronium tetrachlorohydrex glycinate, zinc phenosulfonate, chlorhexidine, hexetidine, zinc citrate, 2,4,4'-trichloro-2'-hydroxydiphenyl ether (triclosan) and 3,4,4'-trichlorocarbanilide (triclocarbon). Amounts of the anti-microbials may be utilized at levels from about 0.0001 % to about 15 %, preferably from about 0.1 % to about 5 % by weight.

Compositions of the present invention may include vitamins. Illustrative vitamins are Vitamin A (retinol), Vitamin B₂, Vitamin B₆, Vitamin C, Vitamin E and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present in compositions according to the present invention may range from 0.001 %to 10 %, preferably from 0.01 % to 1 %, optimally from 0.1 % to 0.5 % by weight.

A variety of herbal extracts may optionally be included in compositions of this invention. Illustrative are green tea, chamomile, licorice and extract combinations thereof. The extracts may either be water soluble or water-insoluble carried in a solvent which respectively is hydrophilic or hydrophobic. Water and ethanol are the preferred extract solvents.

Colorants, fragrances, opacifiers and abrasives may also be included in compositions of the present invention. Each of these substances may range from about 0.05 % to about 5 %, preferably between 0.1 % and 3 % by weight.

Compositions of the present invention will contain water in amounts ranging from about 10 % to about 92 %, preferably from about 30 % to about 70 %, optimally from about 40 % to about 60 % by weight.

Hydrophobic emollients are delivered to the shaving surface by the composition in average droplet size ranging from about 0.00001 to about 350, more preferably from about 0.0001 to about 5, optimally from about 0.001 to about 1 microns in diameter.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material ought to be understood as modified by the word "about".

All documents referred to herein, including patents, patent applications and printed publications, are hereby incorporated by reference in their entirety in this disclosure.

### Examples

The following examples will more fully illustrate the embodiments of this invention. All parts, percentages and proportions referred to herein and in the appended claims are by weight unless otherwise illustrated.

### Examples 1-8

Shaving creams according to the present invention are detailed in the Table below.

| Ingredient | Example (Weight %) | | | | | | | |
|---|---|---|---|---|---|---|---|---|
| | 1 | 2 | 3 | 4 | 5 | 6 | 7 | 8 |
| Sunflower Seed Oil | 24.0 | 24.0 | 16.0 | 16.0 | 12.0 | 12.0 | 19.5 | 19.5 |
| Lanolin Alcohol/ Cholesterol | 1.0 | 1.0 | 1.0 | 1.0 | 1.0 | 1.0 | 1.0 | 1.0 |
| Cocoamide Monoethanol -amide | ' 1.8 | 1.8 | 1.8 | 2.5 | 2.5 | 2.5 | 2.5 | 2.5 |
| Myristyl Alcohol | 1.9 | 1.9 | 1.9 | 2.8 | 1.9 | 2.8 | 2.0 | 3.0 |
| Lauryl Alcohol | -- | -- | -- | -- | 0.9 | -- | 1.0 | -- |
| Petrolatum | 3.5 | 3.5 | 3.5 | 3.5 | 4.5 | 4.5 | 1.5 | 7.5 . |
| Glycerol | 5.4 | 5.4 | 5.4 | 5.4 | 5.4 | 5.4 | 5.4 | 5.4 |
| Cocoamido-propyl Betaine | 3.2 | 6.3 | 3.2 | 6.3 | 3.2 | 3.2 | 3.2 | 3.2 |
| Sodium Laureth Sulphate | 6.3 | 3.2 | 6.3 | 3.2 | 6.3 | 6.3 | 6.3 | 6.3 |
| Guar Hydroxy-propyl Trimonium Chloride | 0.1 | 0.1 | 0.3 | 0.3 | 0.3 | 0.3 | 0.4 | 0.5 |
| Isopropyl Palmitate | 1.4 | 1.4 | 1.4 | 1.4 | 2.7 | 2.7 | 2.7 | 2.7 |
| Tetrasodium EDTA | 0.02 | 0.02 | 0.02 | 0.02 | 0.02 | 0.02 | 0.02 | 0.02 |
| DMDM Hydantoin | 0.05 | 0.05 | 0.05 | 0.05 | 0.05 | 0.05 | 0.05 | 0.05 |
| Etidronic Acid | 0.02 | 0.02 | 0.02 | 0.02 | 0.02 | 0.02 | 0.02 | 0.02 |
| Fragrance | 1.0 | 1.0 | 1.0 | 1.0 | 1.0 | 1.0 | 1.0 | 1.0 |
| Dimethyl Ether | 3.0 | 2.0 | 2.0 | 3.0 | 2.0 | 1.0 | 2.0 | 2.0 |
| Propane | 0.5 | 0.5 | 0.5 | 0.5 | 1.5 | 0.5 | 0.5 | 2.5 |
| Isobutane | 2.5 | 0.5 | 2.5 | 0.5 | 2.5 | 3.5 | 2.0 | 0.5 |
| Water | to 100 | to 100 | to 100 | to 100 | to 100 | to 100 | to 100 | to 100 |

The shaving creams outlined in the Table are packaged in an aerosol metal can fitted with a conventional aerosol spray head with nozzle. Prior to use, instructions on the can request a user to thoroughly shake the combination before expressing the foam by pressing downward on the spray head. After the cream has been placed on a human face, underarm or legs, a razor is applied over the skin area to remove hair by cutting. These formulations are particularly suitable for legs and underarm.

### Example 9

This Example evaluates the effect of different propellant mixtures on the quality of the foam. The formula detailed in the Table below was utilized for evaluations under this Example.

| INGREDIENT | WEIGHT % |
|---|---|
| Sunflower Seed Oil | 21.3 |
| Petrolatum | 5.7 |
| Glycerin | 3.7 |
| Sodium Lauryl Ether Sulphate (3 EO) | 6.6 |
| Cocoamidopropyl Betaine | 3.3 |
| Water | Balance |

The formula of shaving cream outlined above was packaged in aerosol metal canisters fitted with a conventional aerosol spray head with nozzle. Each canister was also filled with a propellant in equal weight throughout. Identity of the propellant is found in the Table below. Samples were evaluated in a Compression (Squeeze) Test and measured for the Liquid Fraction of the foam.

### Measurement of Liquid Fraction of Foam

Liquid volume fractions of foams were obtained by weighing a known volume of foam. The weight of the foam essentially can all be attributed to its liquid fraction as the remaining volume of foam consists of air. To measure the liquid fraction, foam was expelled from a sample canister into a plastic Petri dish having a volume of 5.2 ml. The level of the foam was carefully adjusted to the height of the dish with a metal spatula so that the foam was contained within the volume of the dish. The weight of the dish with the foam was recorded. After subtracting the weight of the dish, the weight of the foam was used to calculate the liquid phase volume. The weight of the foam is divided by the density of the formulation to obtain the volume of the liquid portion of the formula. The liquid fraction is then the volume of the liquid divided by the total volume of foam (in these tests, 5.2 ml). Samples were measured in triplicate.

### Rheology of Foams - Compression (Squeeze) Test

The Compression Test measures the force required to compress the foam generated from a formulation expelled from a canister. Measurements were performed using a Rheometric Scientific ARES controlled strain rheometer (SR-5, Rheometric Scientific, Piscataway, NJ). The rheometer was set up with parallel plates 50 mm in diameter with a gap of 2.0 mm between the plates. Foams were loaded between the parallel plates and then tests were formed using programmed extension mode which subjects the foams to an axial deformation applied at a programmed rate. The foam samples were compressed at controlled rate of 0.25 mm/second, brought back to initial state at a rate of 0.33 mm/sec and then extended at a rate of 0.25 mm/sec. Tests were performed at 25°C. The output is the normal force (grams) associated with the compression and extension of the foams.

Higher force values reflect a more rigid, better foam quality. When product is expelled from a canister, some is in gaseous form and some extrudes as a liquid. The greater the amount of liquid, the lower the foam quality.

| | | Propellant | | Liquid Fraction | | | Compression Test | | |
|---|---|---|---|---|---|---|---|---|---|
| Sample | Can | DME | A70* | % w/w | Average % | Std dev % | Normal Force (g) | Average | Std dev |
| A | 1 | 100 | 0 | 25.93 | 26.39 | 1.94 | 111 | 210.3 | 117.5 |
| | 2 | | | 24.72 | | | 180 | | |
| | 3 | | | 28.51 | | | 340 | | |
| B | 1 | 80 | 20 | 16.97 | 16.96 | 0.38 | 502 | 431.0 | 67.0 |
| | 2 | | | 17.33 | | | 369 | | |
| | 3 | | | 16.57 | | | 422 | | |
| C | 1 | 66 | 33 | 18.05 | 17.14 | 0.85 | 419 | 435.7 | 36.9 |
| | 2 | | | 17.00 | | | 478 | | |
| | 3 | | | 16.37 | | | 410 | | |
| D | 1 | 33 | 66 | 5.53 | 10.64 | 4.60 | 500 | 517.3 | 28.3 |
| | 2 | | | 11.94 | | | 550 | | |
| | 3 | | | 14.45 | | | 502 | | |
| E | 1 | 20 | 80 | 4.24 | 7.36 | 3.91 | 505 | 514.0 | 50.1 |
| | 2 | | | 11.75 | | | 568 | | |
| | 3 | | | 6.08 | | | 469 | | |
| F | 1 | 0 | 100 | 5.24 | 5.65 | 0.74 | 539 | 499.0 | 45.2 |
| | 2 | | | 6.51 | | | 450 | | |
| | 3 | | | 5.20 | | | 508 | | |

| | | | | | | | | | |
|---|---|---|---|---|---|---|---|---|---|
| * Propane/Isobutane mixture | | | | | | | | | |

Sample A was found to deliver a dead non-springy foam. This is evidenced by the relatively low compression test average of 210.3 g force. Samples B to E were able to be worked during hand washing and did not break into undesirable smaller segments of foam. The working ability is attributed to the relatively high level of liquid fraction. By contrast, Sample F was extremely fluffy, containing too much air and did not wash-off well from the skin. It is evident that such as in Samples B through E combinations of dimethyl ether and hydrocarbon are necessary to obtain a satisfactory shaving cream product.

## Claims

1. A shaving cream composition comprising:
(i) from 1 % to 20 % of a propellant comprising dimethyl ether and hydrocarbons in a relative weight ratio of 5:1 to 1:5;
(ii) from 0.5 % to 25 % of an anionic surfactant;
(iii) from 0.05 % to 20 % of a C₁₂-C₂₄ fatty alcohol;
(iv) from 5 % to 40 % of a hydrophobic emollient;
wherein a total weight ratio of all hydrophobic emollients to all anionic surfactants present in the composition ranges from 50:1 to greater than 1:1.

2. The composition according to claim 1 wherein the hydrophobic emollient is selected from vegetable oil, petrolatum, a lanolin oil and combinations thereof.

3. The composition according to claim 2 wherein the vegetable oil is selected from sunflower seed oil, soybean oil and mixtures thereof.

4. The composition according to any of the preceding claims wherein the ratio of dimethyl ether to hydrocarbon ranges from 2:1 to 1:2.

5. The composition according to any of the preceding claims wherein the composition further comprises an amphoteric surfactant.

6. The composition according to any of the preceding claims wherein the total weight ratio of all hydrophobic emollients to all anionic surfactants ranges from 5:1 to 1.5:1.

7. The composition according to any of the preceding claims further comprising from 0.5 % to 30 % of a hydrophilic emollient.

8. The composition according to claim 7 wherein the hydrophilic emollient is glycerol.

9. The composition according to claim 7 wherein a weight ratio of all the hydrophobic to hydrophilic emollients ranges from 20:1 to greater than 1:1.

10. A method of shaving human skin comprising:
(a) providing a shaving cream composition comprising:
(i) from 1 % to 20 % of a propellant comprising dimethyl ether and hydrocarbons in a relative weight ratio of 5:1 to 1:5;
(ii) from 0.5 % to 25 % of an anionic surfactant;
(iii) from 0.05 % to 20 % of a C₁₂-C₂₄ fatty alcohol,
(iv) from 5 % to 40 % of a hydrophobic emollient;
wherein a total weight ratio of all hydrophobic emollients to all anionic surfactants present in the composition ranges from 50:1 to greater than 1:1;
(b) placing the shaving cream composition up a human skin; and
(c) applying a razor to an area of the skin occupied by the shaving cream composition and cutting hair therefrom.

## Patentansprüche

1. Rasiercremezusammensetzung, umfassend:
(i) 1% bis 20% eines Treibmittels, umfassend Dimethylether und Kohlenwasserstoffe in einem relativen Gewichtsverhältnis von 5:1 bis 1:5;
(ii) 0,5% bis 25% eines anionischen Tensids;
(iii) 0,05% bis 20% eines C₁₂-C₂₄-Fettalkohols;
(iv) 5% bis 40% eines hydrophoben Erweichungsmittels;
worin ein Gesamtgewichtsverhältnis von allen hydrophoben Erweichungsmitteln zu allen anionischen Tensiden, die in der Zusammensetzung vorliegen, im Bereich von 50:1 bis größer als 1:1 liegt.

2. Zusammensetzung nach Anspruch 1, wobei das hydrophobe Erweichungsmittel aus Pflanzenöl, Petrolatum, einem Lanolinöl und Kombinationen davon ausgewählt ist.

3. Zusammensetzung nach Anspruch 2, wobei das Pflanzenöl aus Sonnenblumenöl, Sojaöl und Gemischen davon ausgewählt ist.

4. Zusammensetzung nach einem der vorangehenden Ansprüche, wobei das Verhältnis von Dimethylether zu Kohlenwasserstoff im Bereich von 2:1 bis 1:2 liegt.

5. Zusammensetzung nach einem der vorangehenden Ansprüche, wobei die Zusammensetzung weiterhin ein amphoteres Tensid umfasst.

6. Zusammensetzung nach einem der vorangehenden Ansprüche, wobei das Gesamtgewichtsverhältnis von allen hydrophoben Erweichungsmitteln zu allen anionischen Tensiden im Bereich von 5:1 bis 1,5:1 liegt.

7. Zusammensetzung nach einem der vorangehenden Ansprüche, weiterhin umfassend 0,5% bis 30% eines hydrophilen Erweichungsmittels.

8. Zusammensetzung nach Anspruch 7, wobei das hydrophile Erweichungsmittel Glycerin darstellt.

9. Zusammensetzung nach Anspruch 7, wobei das Gewichtsverhältnis von den gesamten hydrophoben zu hydrophilen Erweichungsmitteln im Bereich von 20:1 bis größer als 1:1 liegt.

10. Verfahren zum Rasieren menschlicher Haut, umfassend:
(a) Bereitstellen einer Rasiercremezusammensetzung, umfassend:
(i) 1% bis 20% eines Treibmittels, umfassend Dimethylether und Kohlenwasserstoffe in einem relativen Gewichtsverhältnis von 5:1 bis 1:5;
(ii) 0,5% bis 25% eines anionischen Tensids;
(iii) 0,05% bis 20% eines C₁₂-C₂₄-Fettalkohols;
(iv) 5% bis 40% eines hydrophoben Erweichungsmittels;
worin ein Gesamtgewichtsverhältnis von allen hydrophoben Erweichungsmitteln zu allen anionischen Tensiden, die in der Zusammensetzung vorliegen, im Bereich von 50:1 bis größer als 1:1 liegt.
(b) Anordnen der Rasiercremezusammensetzung auf menschlicher Haut und
(c) Anwenden eines Rasierapparats auf eine Fläche der Haut, die von der Rasiercremezusammensetzung eingenommen wird, und Schneiden von Haaren davon.

## Revendications

1. Composition de crème de rasage comprenant :
(i) de 1 % à 20 % d'un propulseur comprenant de l'éther diméthylique et des hydrocarbures en un rapport en poids relatif de 5:1 à 1:5 ;
(ii) de 0,5 % à 25 % d'un tensioactif anionique ;
(iii) de 0, 05 % à 20 % d'un alcool gras en C₁₂ à C₂₄ ;
(iv) de 5 % à 40 % d'un émollient hydrophobe ;
dans laquelle un rapport en poids total de tous les émollients hydrophobes à tous les tensioactifs anioniques présents dans la composition varie de 50:1 à plus de 1:1.

2. Composition selon la revendication 1 dans laquelle l'émollient hydrophobe est choisi parmi une huile végétale, de la vaseline, de l'huile de lanoline et des combinaisons de celles-ci.

3. Composition selon la revendication 2, dans laquelle l'huile végétale est choisie parmi l'huile de tournesol, l'huile de soja et des mélanges de ceux-ci.

4. Composition selon l'une quelconque des revendications précédentes dans laquelle le rapport de l'éther diméthylique à l'hydrocarbure varie de 2:1 à 1:2.

5. Composition selon l'une quelconque des revendications précédentes, dans laquelle la composition comprend en outre un tensioactif amphotère.

6. Composition selon l'une quelconque des revendications précédentes, dans laquelle le rapport en poids total de tous les émollients hydrophobes à tous les tensioactifs anioniques varie de 5:1 à 1,5:1.

7. Composition selon l'une quelconque des revendications précédentes, comprenant en outre de 0,5 % à 30 % d'un émollient hydrophile.

8. Composition selon la revendication 7, dans laquelle l'émollient hydrophile est la glycérine.

9. Composition selon la revendication 7, dans laquelle un rapport en poids de tous les émollients hydrophobes aux émollients hydrophiles varie de 20:1 à plus de 1:1.

10. Procédé de rasage de la peau humaine comprenant :
(a) l'apport d'une composition de crème de rasage comprenant .
(i) de 1 % à 20 % d'un propulseur comprenant de l'éther diméthylique et des hydrocarbures en un rapport en poids relatif de 5:1 à 1:5 ;
(ii) de 0,5 % à 25 % d'un tensioactif anionique ;
(iii) de 0,05 % à 20 % d'un alcool gras en C₁₂ à C₂₄ ;
(iv) de 5 % à 40 % d'un émollient hydrophobe ;
dans laquelle un rapport en poids total de tous les émollients hydrophobes à tous les tensioactifs anioniques présents dans la composition varie de 50:1 à plus de 1:1.
(b) l'application de la composition de crème de rasage sur la peau humaine ; et
(c) le placement d'un rasoir sur une zone de la peau occupée par la composition de crème de rasage et la coupe des poils en cet endroit.
